# EUROPÄISCHE PATENTSCHRIFT

(11) **EP 1 088 514 B1**
(45) Veröffentlichungstag und Bekanntmachung des Hinweises auf die Patenterteilung: **21.02.2007**
(21) Anmeldenummer: 00203364.5
(22) Anmeldetag: 21.09.2000
(51) Int. Cl.: A61B 5/06

(54) **Verfahren und Anordnung zur Bestimmung der Position eines medizinischen Instruments**
Method and device for determining position of a medical instrument
Procédé et dispositif pour déterminer la position d'un instrument médical

(30) Priorität: 30.09.1999 DE 19946948
(43) Veröffentlichungstag der Anmeldung: 04.04.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Rasche, Volker, Dr., 52064 Aachen (DE)
(74) Vertreter: Volmer, Georg

(56) Entgegenhaltungen:
- WO-A-97/29682
- WO-A-99/43253
- US-A- 5 730 129

## Beschreibung

Die Erfindung betrifft ein Verfahren und eine Anordnung zur Bestimmung der Position eines in den Körper eines Patienten eingeführten medizinischen Instruments relativ zu einem sich periodisch bewegenden Körperorgan.

Klinische Anwendungen stellen immer höhere Anforderungen an die Genauigkeit der Positionsbestimmung eines in den Körper eines Patienten eingeführten medizinischen Instruments. Insbesondere das immer stärker werdende Interesse an minimal-invasiven Behandlungsmethoden zur Behandlung von Herzkrankheiten erfordert die Entwicklung von Verfahren und Anordnungen, die es dem Arzt ermöglichen, ein medizinisches Instrument an eine genau vorbestimmte Position innerhalb oder außerhalb des Herzens zu führen. Beispielsweise ist es bei der Direkten Myokardialen Revaskularisation erforderlich, ein Katheter mit einer kleinen Bohrspitze an mehrere vorbestimmte Positionen an der Ventrikelwand zu bringen, um dort kleine Löcher in die Ventrikelwand bohren oder um an solchen Stellen ein Medikament direkt in die Ventrikelwand einzubringen.

Bei einem aus Gepstein et al., "A Novel Method for Nonfluoroscopic Catheter-Based Electroanatomical Mapping of the Heart", Circulation 1997; 95:1611-1622 bekannten Verfahren wird die Position eines in den Körper eingeführten Katheters dadurch gemessen, dass an dem Katheter eine elektromagnetische Sendevorrichtung, beispielsweise eine HF-Spule, und außerhalb des Körpers eine korrespondierende elektromagnetische Empfangsvorrichtung, beispielsweise mehrere HF-Empfangsspulen, angeordnet sind zum Empfang der von der Sendevorrichtung ausgesendeten Signale. Dadurch kann zwar die Position des Katheters bzw. der Katheterspitze verhältnismäßig genau relativ zum Koordinatensystem der Empfangsvorrichtung, d.h. die Position des Katheters im Raum bestimmt werden, eine Bestimmung der Position des Katheters relativ zu der umliegenden Anatomie, beispielsweise relativ zum Herzen, ist damit allerdings nicht möglich. Dazu müßten während der Behandlung zusätzlich Röntgenfluoreskopieaufnahmen gemacht werden, die eine Verfolgung des Katheters in ständig neu aufgenommenen Röntgenbildern ermöglichen würden. Derartige Fluoreskopieverfahren sind jedoch einerseits verhältnismäßig aufwendig und liefern auch nicht die geforderte Genauigkeit bei der Positionsbestimmung des medizinischen Instruments. Andererseits führt eine kontinuierliche Röntgenbilderfassung während der Behandlung des Patienten zu einer zusätzlichen Belastung mit Röntgenstrahlung. Hinzu kommt das weitere Problem, dass die das medizinische Instrument umgebende Anatomie während der Behandlung nicht in Ruhe ist, sondern sich bewegt, insbesondere nach einer periodischen Bewegung. Dies gilt vor allem für das Herz, das eine periodische Eigenbewegung ausführt, nämlich sich zusammenzieht während der Systole und sich ausdehnt während der Diastole, und zudem aufgrund der Atmung des Patienten einer zusätzlichen nahezu periodischen Bewegung ausgesetzt ist. Neben dem Herzen gilt dies auch, teilweise in geringerem Ausmaß, für andere Körperorgane wie das Gehirn, den Magen und die Leber, die ebenfalls durch die Herz- und die Atembewegung bewegt werden.

Aus US-A-5 730 129 ist ein Verfahren zur Bestimmung der Position eines in den Körper eines Patienten eingeführten medizinischen Instruments relativ zu einem sich periodisch bewegenden Körperorgan bekannt, das die folgenden Verfahrensschritte aufweist :
a) Bestimmung der Raumposition des medizinischen Instruments bei gleichzeitiger Erfassung eines im Zusammenhang mit der periodischen Bewegung des Körperorgans stehenden periodischen Bewegungssignals,
b) Auswahl eines Bilddatensatzes aus einer Bilddatenbank, welche präoperativ zeitgleich mit demselben Bewegungssignal erfaßte, einzelnen Bewegungsphasen zugeordnete Bilddatensätze des Körperorgans enthält, derart, dass derjenige Bilddatensatz ausgewählt wird, welcher der Bewegungsphase bei der Bestimmung der Raumposition des medizinischen Instruments zugeordnet ist, und
c) Bestimmung der Position des medizinischen Instruments relativ zu dem Körperorgan durch Umrechnung des ausgewählten Bilddatensatzes in ein verzerrtes Bild, in dem das medizinische Instrument dargestellt wird.

Der vorliegenden Erfindung liegt demnach die Aufgabe zugrunde, ein Verfahren und eine Anordnung anzugeben, die die Bestimmung der Position eines in den Körper eines Patienten eingeführten medizinischen Instruments relativ zu einem sich periodisch bewegenden Körperorgan mit möglichst hoher Genauigkeit ermöglichen.

Diese Aufgabe wird erfindungsgemäß durch ein Verfahren gemäß Anspruch 1 und eine Anordnung gemäß Anspruch 10 gelöst.

Der Erfindung liegt dabei die Erkenntnis zugrunde, dass die periodische Bewegung des Körperorgans, relativ zu dem die Position des medizinischen Instruments bestimmt werden soll, zur Positionsbestimmung mit herangezogen werden kann. Dazu wird ein im Zusammenhang mit der periodischen Bewegung des Körperorgans stehendes periodisches Bewegungssignal gemessen, beispielsweise ein von der Atembewegung des Patienten abhängiges Atembewegungssignal oder ein mit der Herzbewegung zusammenhängendes Elektrokardiogramm, während mittels einer Meßanordnung, beispielsweise mittels der bekannten elektromagnetischen Meßeinrichtung, die Raumposition des medizinischen Instruments und einer Referenzprobe bestimmt wird. Die Referenzprobe ist dabei außerhalb des Körpers des Patienten, beispielsweise an dessen Körperoberfläche oder am Patiententisch, angeordnet und derart ausgestaltet, dass ihre Position mittels der Positionsmeßeinheit bestimmt werden kann. Es können auch zwei Referenzproben vorgesehen sein, von denen eine am Körper des Patienten und die zweite am Patiententisch angeordnet sind.

Bereits vor dem medizinischen Eingriff ist mittels einer Bilddatenerfassungseinheit, beispielsweise mittels einer Magnetresonanztomographieeinheit, eines Computertomographen, einer Ultraschall- oder Röntgeneinrichtung, eine Bilddatenbank aufgebaut worden, in die einzelne 3D-Bilddatensätze aufgenommen sind, die zeitgleich mit demselben periodischen Bewegungssignal erfaßt wurden, das auch bei der intraoperativen Bestimmung der Raumpositionen erfaßt wird, wobei jeder einzelne 3D-Bilddatensatz einer einzelnen Bewegungsphase innerhalb einer Periode des Bewegungssignals zugeordnet ist. Außerdem muß präoperativ auch die Referenzprobe bereits an ihrem späteren Anbringungsort angeordnet sein und ihre Position relativ zu den 3D-Bilddatensätzen bestimmt werden. Dies kann beispielsweise dadurch geschehen, dass die Referenzprobe so ausgestaltet ist, dass sie bei der Bilddatenerfassung mit erfaßt und in den einzelnen 3D-Bilddatensätzen erkennbar ist. Eine andere Möglichkeit besteht darin, dass die Position der Referenzprobe relativ zur Bilddatenerfassungseinheit bestimmt wird, z.B. indem mittels der bekannten Positionsmeßeinheit die Position der Bilddatenerfassungseinheit und der Referenzprobe bestimmt wird.

Anhand der Bewegungsphase bei der Bestimmung der Raumposition des medizinischen Instruments und der Referenzprobe wird nun (intraoperativ) aus der Bilddatenbank derjenige 3D-Bilddatensatz ausgesucht, welcher derselben Bewegungsphase zugeordnet ist. Das Bewegungssignal, genauer gesagt die einzelne Bewegungsphase, stellt somit quasi die Verknüpfung her zwischen der intraoperativ ermittelten Raumposition des medizinischen Instruments und dem die Information über die Lage der Anatomie in derselben Bewegungsphase enthaltenden präoperativ ermittelten 3D-Bilddatensatz. Da in diesem 3D-Bilddatensatz auch die Position der Referenzprobe relativ zu dem Körperorgan bekannt ist, und die aktuelle Raumposition der Referenzprobe gemessen wurde, kann daraus eine Umrechnungsformel ermittelt werden, durch die die gemessene Raumposition des medizinischen Instruments auf einfache Weise, beispielsweise mittels einfacher Koordinatentransformation, in dessen Position relativ zu dem Körperorgan umgerechnet werden kann. Durch die Erfindung wird es somit ermöglicht, die Position eines in den Körper eines Patienten eingeführten medizinischen Instruments relativ zu einem sich periodisch bewegenden Körperorgan, beispielsweise die Position eines in eine Herzkammer eingeführten Katheters, exakt zu bestimmen und Bewegungen des Instruments zu verfolgen. Der Arzt kann somit das Instrument an genau vorbestimmte Positionen dirigieren und dort die gewünschten Eingriffe vornehmen.

Vorteilhafte Ausgestaltungen des erfindungsgemäßen Verfahrens bzw. der erfindungsgemäßen Anordnung ergeben sich aus den weiteren Ansprüchen.

Die Entscheidung, welches Bewegungssignal aufgezeichnet und bei dem erfindungsgemäßen Verfahren verwendet werden soll, hängt insbesondere davon ab, welcher Bewegung das in den Körper eingeführte medizinische Instrument selbst ausgesetzt ist, bzw. welche Bewegung das Körperorgan ausführt, innerhalb dessen oder in dessen Umgebung das medizinische Instrument arbeiten soll. Bei einem Herzkatheter wird dies insbesondere die Herzbewegung sein, so dass es sich vorteilhaft anbietet, ein Elektrokardiogramm des Patienten als Bewegungssignal zu erfassen. Bei Eingriffen am Gehirn kommt ebenfalls ein Elektrokardiogramm in Betracht. In einer vorteilhaften Ausgestaltung ist auch vorgesehen, als Bewegungssignal ein von der Atembewegung des Patienten abhängiges Atembewegungssignal zu erfassen.

In einer vorteilhaften Weiterbildung ist vorgesehen, ein solches Atembewegungssignal zusätzlich zu einem anderen Bewegungssignal, beispielsweise dem Elektrokardiogramm, zu erfassen und dazu zu verwenden, bei der Bestimmung der Position des medizinischen Instruments gemäß dem oben beschriebenen Verfahren auch die Bewegungen der Anatomie und ggf. des medizinischen Instruments aufgrund der Atmung zu berücksichtigen und entsprechend zu korrigieren. Bei dieser Weiterbildung wird eine noch größere Genauigkeit der Positionsbestimmung erreicht.

In einer vorteilhaften Ausgestaltung ist zudem vorgesehen, auch während der Bestimmung der Raumposition des medizinischen Instruments und der Referenzprobe einen 3D-Bild datensatz zu erfassen, was beispielsweise mittels eines Echtzeit-3D-Ultraschallverfahrens möglich ist, und bei der Bestimmung der Position des medizinischen Instruments relativ zu dem Körperorgan zu verwenden und/oder in die Bilddatenbank aufzunehmen. Dadurch kann ggf. eine weitere Erhöhung der Genauigkeit der Positionsbestimmung erreicht werden. Außerdem stehen dadurch Informationen über einen Echtzeit-3D-Bilddatensatz zur Verfügung, die bei der Erstellung eines Bildes des Umgebungsbereichs des medizinischen Instruments aus dem ausgewählten 3-D-Bilddatensatz mit verwendet werden, welches Bild dann auf einer Anzeigevorrichtung darstellbar ist und in welches die Position des medizinischen Instruments eingeblendet werden kann, was bei einer weiteren vorteilhaften Ausgestaltung vorgesehen ist.

Die Erfindung wird nachfolgend anhand der Zeichnungen näher erläutert. Es zeigen:
- Fig. 1: ein Blockschaltbild zur Erläuterung des erfindungsgemäßen Verfahrens,
- Fig. 2: einen Ablaufplan der einzelnen Verfahrensschritte,
- Fig. 3: mehrere Zeitdiagramme zur Erläuterung einzelner Verfahrensschritte,
- Fig. 4: die Darstellung eines menschlichen Herzens mit eingeblendeter Katheterspitze,
- Fig. 5: ein Blockschaltbild einer weiteren Ausführungsform der Erfindung und
- Fig. 6: ein Zeitdiagramm eines Atembewegungssignals.

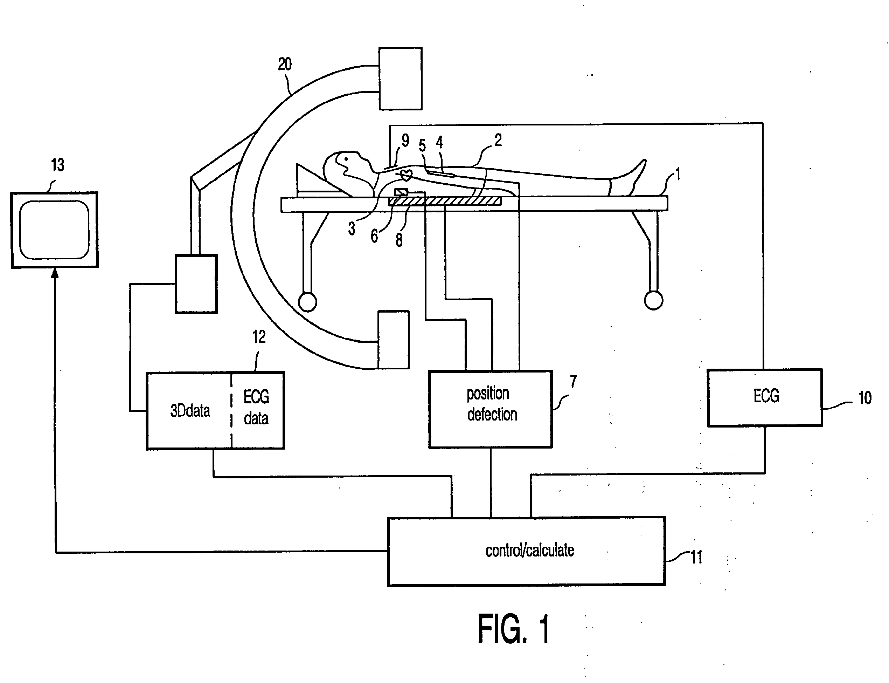

Das in Fig. 1 dargestellte Blockschaltbild zeigt einen auf einem Patiententisch 1 liegenden Patienten 2, an dessen symbolisch angedeutetem Herzen 3 eine Behandlung mittels eines in den Körper eingeführten Katheters 4 durchgeführt werden soll. An der Spitze des Katheters ist neben dem nicht gezeigten Behandlungsinstrument, z.B. einem kleinen Laser oder Bohrer, eine Hochfrequenzspule (HF-Spule) 5 zum Aussenden von HF-Signalen angeordnet. Zum Empfang der von der HF-Spule 5 ausgesendeten Signale ist unterhalb des Patienten 2, beispielweise in den Patiententisch 1 integriert, eine korrespondierende HF-Empfangseinheit 8, z.B. ein mindestens drei nebeneinander angeordnete HF-Empfangsspulen aufweisendes Tableau, angeordnet. Der Katheter 4 mit der HF-Sendespule 5 und die HF-Empfangseinheit 8 sind mit einer HF-Verarbeitungseinheit 7 verbunden, die zusammen mit der HF-Sendespule 5 und der HF-Empfangseinheit 8 eine Positionsmeßeinheit bildet. In der HF-Verarbeitungseinheit 7 wird einerseits die Aussendung der HF-Signale von der Spule 5 gesteuert, andererseits werden dort die von der HF-Empfangseinheit 8 empfangenen Signale ausgewertet und daraus die Position des Katheters 4 in einem ortsfesten Koordinatensystem, beispielsweise im Koordinatensystem der HF-Empfangseinheit 8, berechnet, woraus die Position des Katheters 4 im Raum bestimmt werden kann.

Weiterhin ist mit der HF-Verarbeitungseinheit 7 eine Referenzprobe 6 verbunden, die ebenfalls HF-Sendemittel, beispielsweise eine HF-Sendespule, zum Aussenden von HF-Signalen aufweisen, die ebenfalls mittels der HF-Empfangseinheit 8 empfangen werden können und woraus sich die Position der Referenzprobe 6 im Raum bestimmen lassen. Die Referenzprobe 6 ist im gezeigten Fall ortsfest am Patiententisch 1 angeordnet, kann aber auch fest an der Körperoberfläche des Patienten 2 angeordnet sein, sofern sich dieser während der Behandlung nicht bewegt.

Im Brustbereich des Patienten 2 ist eine Elektrodenvorrichtung 9 angeordnet, die mit einer Elektrokardiographieeinheit 10 verbunden ist zur Messung eines Elektrokardiogramms des Patienten 2 während der Behandlung.

Die HF-Verarbeitungseinheit 7 und die Elektrokardiographieeinheit 10 sind mit einer Steuer- und Recheneinheit 11 verbunden, welche die genannten Einheiten steuert und die davon gelieferten Daten weiterverarbeitet. Außerdem ist mit der Steuer- und Recheneinheit 11 eine Speichereinheit 12 verbunden, in der präoperativ erfaßte 3D-Bilddatensätze des Patienten 2, im vorliegenden Fall dessen Herzregion, gespeichert sind. Diese 3D-Bilddatensätze können je nach gewünschter Information und je nach Anwendung einer oder mehreren medizinischen bildgebenden Einrichtungen (Bilddatenerfassungseinheit 20) wie einer Röntgeneinrichtung oder einer Magnetresonanz-Tomographieeinrichtung geliefert worden sein. Voraussetzung für die Verwertbarkeit dieser 3D-Bilddatensätze bei dem vorgeschlagenen Verfahren ist allerdings, dass bei der Erfassung der 3D-Bilddatensätze die Referenzprobe 6 auch schon an ihrem jetzigen Ort angeordnet war und dass deren Position relativ zur Bilddatenerfassungseinheit 20 bekannt ist. Weitere Voraussetzung ist, dass gleichzeitig zur Erfassung der 3D-Bilddatensätze ein Elektrokardiogramm des Patienten erfaßt wurde, so dass jeder 3D-Bilddatensatz einer konkreten Herzbewegungsphase, d.h. einem bestimmten Zeitpunkt innerhalb der Periode des Elektrokardiogramms, zugeordnet werden kann. Die Speichereinheit 12 enthält somit einen vierdimensionalen Datensatz, nämlich zu jeder Herzbewegungsphase innerhalb der Periode des Elektrokardiogramms einen oder mehrere 3D-Bilddatensätze.

Aus den gelieferten Daten wird nun in der Steuer- und Recheneinheit 11 die Position des Katheters 4 relativ zum Herzen 3 bestimmt, und es kann anhand der vorhandenen 3D-Bilddatensätze ein Bild der den Katheter umgebenden Anatomie ermittelt werden, welches auf einer Anzeigevorrichtung 13 darstellbar ist. Da dies während der Behandlung fortlaufend möglich ist, kann der behandelnde Arzt anhand der dargestellten Bilder, in die jede aktuelle Position des Katheters oder das Katheter selbst eingeblendet werden kann, sehen, wo sich der Katheter gerade befindet. Er kann somit bestimmte Punkte, z.B. innerhalb des Herzens, mit hoher Genauigkeit ansteuern, da gemäß dem vorgeschlagenen Verfahren die Eigenbewegung des Herzens bei der Positionsbestimmung des Katheters relativ zum Herzen mit berücksichtigt wird und nicht zu Fehlergebnissen führen kann.

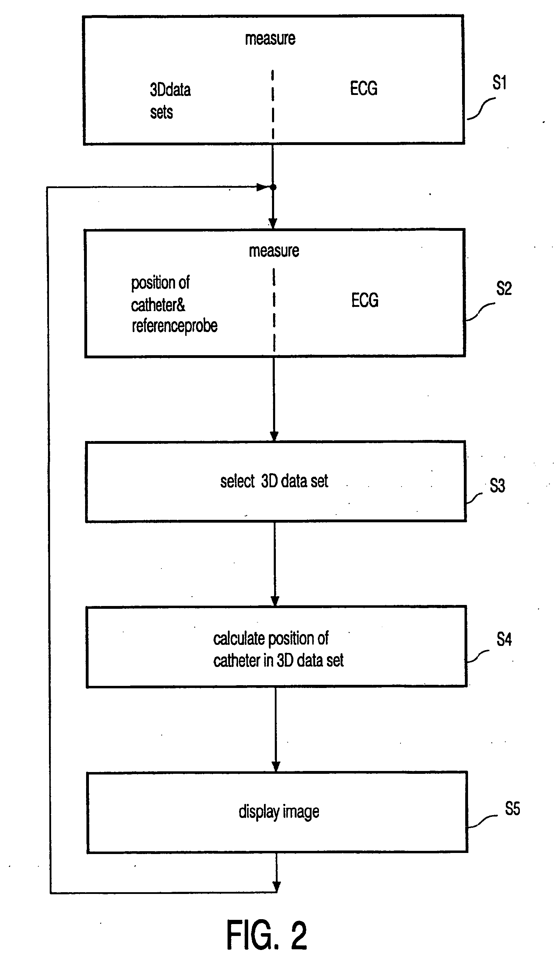

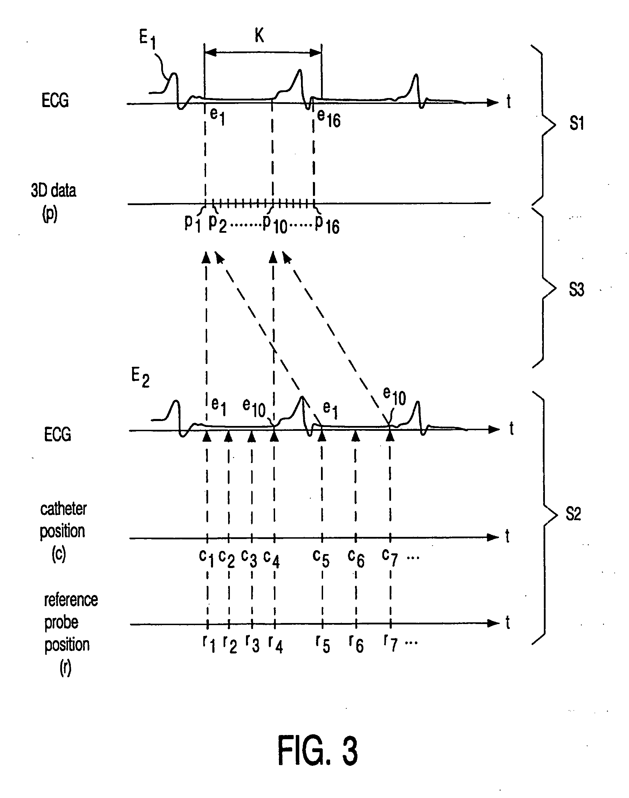

Anhand der Figuren 2 und 3 soll das erfindungsgemäße Verfahren näher erläutert werden. In Fig. 2 sind die einzelnen Verfahrensschritte S1 bis S5 in einem Ablaufplan dargestellt. In dem präoperativ durchzuführenden Verfahrensschritt S1 werden die 3D-Bilddatensätze und gleichzeitig das Elektrokardiogramm des Patienten erfaßt und als Bilddatenbank in der Speichereinheit 12 abgelegt. Bei den nachfolgenden während der Behandlung durchzuführenden Verfahrensschritten wird zunächst im Verfahrensschritt S2 die Raumposition des Katheters und der Referenzprobe mittels der Positionsmeßeinheit gemessen, während gleichzeitig ein Elektrokardiogramm des Patienten geschrieben wird. Anhand der Bewegungsphase des Herzens, d.h. anhand des Zeitpunkts innerhalb einer Periode des Elektrokardiogramms, zu dem diese Positionsmessung erfolgt ist, wird nun im Verfahrensschritt S3 derjenige oder diejenigen 3D-Bilddatensätze aus der Bilddatenbank ausgewählt, die derselben Bewegungsphase, also demselben Zeitpunkt innerhalb der Periode des Elektrokardiogramms zugeordnet sind. Aus der aktuell gemessenen Raumposition des Katheters und der Referenzprobe, dem ausgewählten 3D-Bilddatensatz und der Information über die Position der Referenzprobe relativ zum 3D-Bilddatensatz wird anschließend im Verfahrensschritt S4 die Position des Katheters in dem 3D-Bilddatensatz und damit die Position des Katheters relativ zum Herzen, über das Bildinformationen in dem 3D-Bilddatensatz enthalten sind, umgerechnet. Für diese Umrechnung wird die gemessene Raumposition der Referenzprobe und die Information über die Position der Referenzprobe relativ zum 3D-Bilddatensatz derart ausgenutzt, dass aus den aktuell gemessenen Raumpositionen des Katheters und der Referenzprobe die relative Lage des Katheters zur Referenzprobe ermittelt und dann in den 3D-Bilddatensatz übertragen wird. Schließlich kann aus dem ausgewählten 3D-Bilddatensatz ein Bild des Herzens ermittelt werden, in das die Position des Katheters oder der Katheter selbst eingeblendet ist.

Die Verfahrensschritte S2 bis S5 können fortlaufend während der Behandlung durchgeführt werden, um dem Arzt die Verfolgung des Katheters mit hoher Zuverlässigkeit zu ermöglichen.

Fig. 3 zeigt mehrere Zeitdiagramme, anhand deren die Verfahrensschritte S1 bis S3 näher erläutert werden sollen. Im präoperativ durchzuführenden Verfahrensschritt S1 wird gleichzeitig ein Elektrokardiogramm E₁ und 3D-Bilddatensätze p ermittelt. Diese Ermittlung erfolgt derart, dass jeder einzelnen Bewegungsphase e₁, e₂, ..., e₁₆ innerhalb einer Periode K des Elektrokardiogramms E₁ jeweils ein 3D-Bilddatensatz p₁, p₂,..., p₁₆ zugeordnet wird. Diese präoperativ ermittelten Daten werden als Bilddatenbank abgelegt. In dem während der Behandlung durchzuführenden Verfahrensschritt S2 wird wiederum ein Elektrokardiogramm E₂ geschrieben, wobei gleichzeitig mit der Positionsmeßeinheit die Raumpositionen c des Katheters und die Raumpositionen r der Referenzprobe gemessen werden. Diese Messung der Katheterpositionen c₁, c₂, ...., c₇ und der Referenzprobenpositionen r₁, r₂,..., r₇ kann in gleichen oder in verschiedenen Zeitabständen erfolgen. Jede Positionsmeßung ist jedoch aufgrund der zeitgleichen Erfassung des Elektrokardiogramms E₂ wiederum einer bestimmten Bewegungsphase e innerhalb einer Periode K des Elektrokardiogramms E₂ zuweisbar. Beispielsweise sind im gezeigten Fall die Katheterpositionen c₁ und c₅ sowie die Referenzprobenpositionen r₁ und r₅ in der Bewegungsphase e₁ erfolgt, die Katheterposition c₄, c₇ und die Referenzprobenpositionen r₄, r₇ in der Bewegungsphase e₁₀. Anhand dieser Bewegungsphase wird nun im Verfahrensschritt S3 derjenige 3D-Bilddatensatz aus der Bilddatenbank ausgewählt, der derselben Bewegungsphase dort zugeordnet ist. Im vorliegenden Fall heißt dies, dass anhand der Bewegungsphase e₁ aus der Bilddatenbank der 3D-Bilddatensatz p₁ ausgewählt wird und dass anhand der Bewegungsphase e₁₀ der 3D-Bilddatensatz p₁₀ ausgewählt wird. Es wird somit anhand der jeweiligen Bewegungsphase eine Verknüpfung hergestellt zwischen aktuell gemessener Katheterposition und Referenzprobenposition mit dem präoperativ in derselben Bewegungsphase ermittelten 3D-Bilddatensatz.

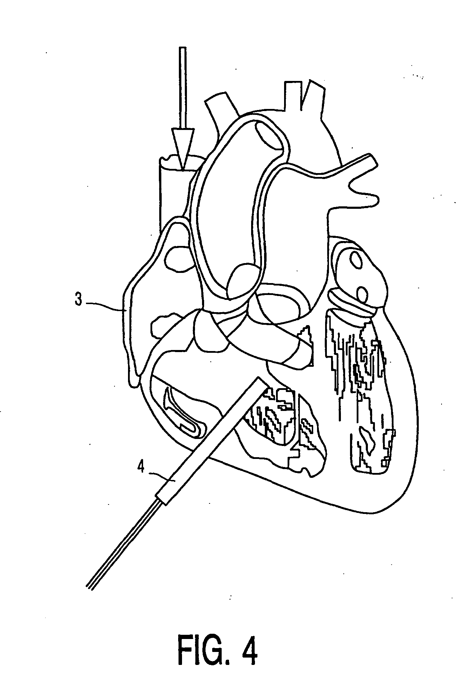

Eine aus einem 3D-Bilddatensatz gewonnene Darstellung des Herzens 3, in die der Katheter 4 eingeblendet ist, ist in Fig. 4 gezeigt.

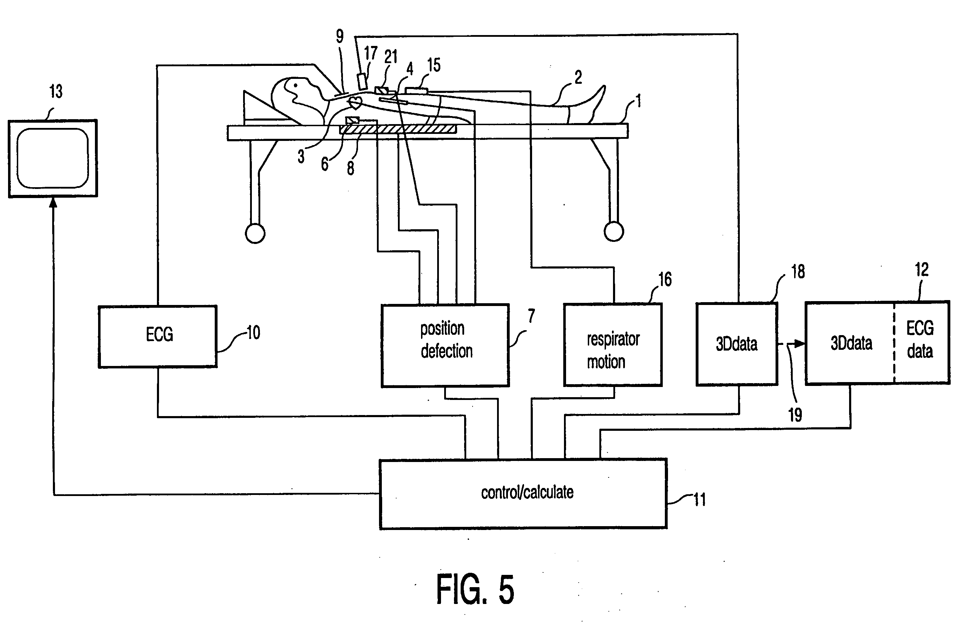

Fig. 5 zeigt eine weitere Ausführungsform der Erfindung in einem Blockschaltbild. Zusätzlich zu den in Fig. 1 gezeigten Mitteln ist bei dieser Ausführungsform eine an der Körperoberfläche des Patienten 2 angebrachte Patienten-Referenzprobe 21 vorgesehen. Diese ist ebenfalls mit der HF-Verarbeitungseinheit 7 verbunden, und deren Position im Raum wird wie die Katheterposition und die Referenzprobenposition laufend ermittelt. Die gemessene Position der Patienten-Referenzprobe 21 kann vorteilhaft dazu benutzt werden, um Bewegungen des Patienten 2 während der Behandlung bei der Bestimmung der Position des Katheters relativ zum Herzen zu berücksichtigen. Dazu wird die relative Lage der Raumpositionen der beiden Referenzproben 6, 21, deren relative Lage im Ausgangszustand . (Ruhezustand des Patienten) bekannt ist, dazu benutzt, das Ausmaß der Positionsveränderung des Patienten während der Behandlung zu bestimmen und bei der weiteren Berechnung zu korrigieren. Die relative Lage der Raumposition des Katheters 4 zur PatientenReferenzprobe 21 wird anschließend dazu benutzt, die Raumposition des Kathters 4 in eine Position relativ zum 3D-Bilddatensatz und damit zum Herzen umzurechnen.

Außerdem ist bei dieser Ausführungsform ein Atembewegungssensor 15 vorgesehen, der die Atembewegung des Patienten 2 überwacht und ein Atembewegungssignal mißt, das an die Atembewegungsmeßeinheit 16 geliefert wird. Dieser Atembewegungssensor 15 kann beispielsweise wie im gezeigten Fall ein im Bauchbereich des Patienten 2 angeordneter durch die Atembewegung elastisch verformbarer Bauchgurt sein, denkbar sind hierfür jedoch auch andere Mittel wie z.B. eine Ultraschalleinrichtung oder eine im Bauchbereich des Patienten angeordnete Widerstandsmeßeinrichtung zur Messung des sich aufgrund der Atmung veränderten elektrischen Widerstands des Patienten 2. Das kontinuierlich während der Messung der Raumpositionen des Katheters und der Referenzproben gemessene Atembewegungssignal wird ebenfalls an die Steuer- und Recheneinheit 11 weitergegeben und dort bei der Berechnung der Position des Katheters relativ zum Herzen mit berücksichtigt. Dies ist deshalb vorteilhaft, weil der Katheter 4 aufgrund der Atembewegung des Patienten ebenfalls im Körper bewegt wird und sich dadurch die relative Lage des Katheters 4 bezüglich der Referenzprobe 6 verändert, nicht aber oder nur in geringem Maße die relative Lage des Katheters gegenüber dem Herzen 3. Ohne Berücksichtigung der Atembewegungsphase bei der Messung der Raumposition des Katheters 4 würde somit die Umrechnung der Raumposition des Katheters 4 in eine Position relativ zum Herzen 3 möglicherweise Fehler ergeben. Ggf. ist auch die Patienten-Referenzprobe 21 ausreichend, um die Funktion der Atembewegungssensors 15 und der Atembewegungsmeßeinheit 16 zu erfüllen, so dass diese Elemente entfallen können.

Weiter ist bei der in Fig. 5 gezeigten Ausführungsform eine Ultraschalleinrichtung 17 vorgesehen, die mit einer Bilddatenerfassungseinheit 18 verbunden ist und mittel der auch während der Behandlung aktuelle 3D-Bilddatensätze in Echtzeit erfaßt werden können. Diese aktuellen 3D-Bilddatensätze können dann über eine Zuleitung 19 den einzelnen Bewegungsphasen zugeordnet und in die Speichereinheit 12 mit aufgenommen werden. Es ist auch eine Weitergabe an die Steuer- und Recheneinheit 11 möglich, wo die aktuellen 3D-Bilddatensätze mit ausgewertet werden. Dies kann zu einer Erhöhung der Genauigkeit der Positionsbestimmung des Katheters 4 und verbesserter Bildqualität bzw. aktuelleren Bildern der den Katheter umgebenden Anatomie führen, da die Erfassung der in der Bild-datenbank gespeicherten 3D-Bilddatensätze präoperativ erfolgte und sich die Anatomie oder deren Lage möglicherweise geringfügig seitdem geändert hat.

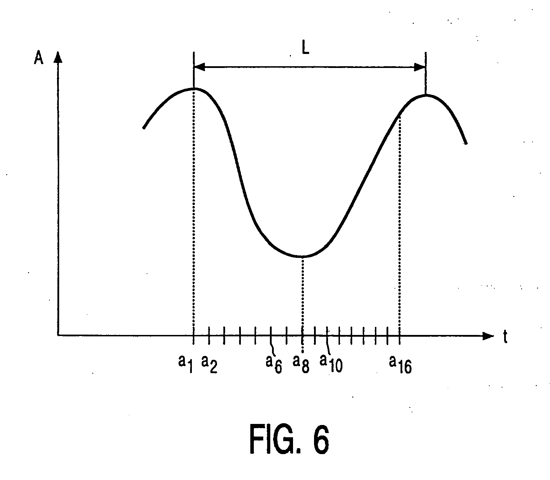

In Fig. 6 ist ein typisches mittels eines Atembewegungssensors 15 gemessenes Atembewegungssignal A über der Zeitachse t dargestellt. Auch ein Atembewegungssignal verläuft im wesentlichen periodisch mit der Periode L und läßt sich ebenfalls in einzelne Atembewegungsphasen a₁, a₂,...., a₁₆ unterteilen.

Dieses Atembewegungssignal A kann, wie anhand von Fig. 5 beschrieben, kontinuierlich und ergänzend zu dem Elektrokardiogramm während der Behandlung gemessen und bei der Berechnung der Position des Katheters relativ zum Herzen berücksichtigt werden.

Dazu wird angenommen, dass sich die Lage der Anatomie im Bereich des Herzens in jeder Atembewegungphase a gegenüber einer bestimmten Referenzlage zu einer bestimmten Referenz-Atembewegungsphase um einen festen Wert verändert. Diese Werte können entweder anhand eines Modells der Anatomie gewonnen oder präoperativ an dem zu behandelnden Patienten gemessen werden. Es kann außerdem vorgesehen werden, ein solches Atembewegungssignal auch schon bei der präoperativen Erfassung der 3D-Bild-datensätze vorzunehmen und die 3D-Bilddatensätze nicht nur den einzelnen Bewegungsphasen des Elektrokardiogramms, sondern zusätzlich auch noch einzelnen Bewegungsphasen der Atembewegung zuzuordnen und entsprechend in der Bilddatenbank einen fünfdimensionalen Datensatz (3D-Bilddatensatz + Herzbewegungsphase + Atembewegungsphase) abzulegen.

Alternativ kann auch vorgesehen sein, die 3D-Bilddatensätze präoperativ nur während bestimmter, bewegungsarmer Atembewegungsphasen, z.B. während der Atembewegungsphasen a₆ bis a₁₀, vorzunehmen, und entsprechend auch während der Behandlung die Raumposition des Katheters nur während derselben Atembewegungsphasen zu ermitteln und auszuwerten.

Statt eines Elektrokardiogramms bei der Erfassung der präoperativen 3D-Bilddatensätze und bei der intraoperativen Messung der Raumposition des Katheters kann auch ein anderes periodisches, im Zusammenhang mit einer periodischen Bewegung eines Körperorgans stehendes Signal, das eine Bewegung der Anatomie und/oder des Katheters im Behandlungsbereich bewirkt, bei dem erfindungsgemäßen Verfahren verwendet werden. So ist es beispielsweise denkbar, bei einer Behandlung im Abdominalbereich des Patienten statt eines Elektrokardiogramms das eben beschriebene Atembewegungssignal (siehe Fig. 6) zu verwenden.

Die anhand der Figuren erläuterten Mittel zur Ermittlung der unterschiedlichen Positionen und Signale sind lediglich beispielhaft zu verstehen. So kann z.B. die Positionsmeßeinheit anders aufgebaut sein, indem z.B. an dem Katheter und an der Referenzprobe jeweils eine Empfangsspuleneinheit und außerhalb des Patienten eine Sendespuleneinheit angeordnet sind. Statt einer Elektrokardiographieeinheit zur Messung eines Elektrokardiogramms können auch andere Mittel wie ein Pulsoxymetrieeinrichtung vorgesehen sein.

## Patentansprüche

1. Verfahren zur Bestimmung der Position eines in den Körper eines Patienten (2) eingeführten medizinischen Instruments (4) relativ zu einem sich periodisch bewegenden Körperorgan (3) mit den Verfahrensschritten:
a) Bestimmung der Raumposition (c, r) des medizinischen Instruments (4) und einer Referenzprobe (6) bei gleichzeitiger Erfassung eines im Zusammenhang mit der periodischen Bewegung des Körperorgans (3) stehenden periodischen Bewegungssignals (E₂),
b) Auswahl eines 3D-Bilddatensatzes (p) aus einer Bilddatenbank, welche präoperativ zeitgleich mit demselben Bewegungssignal (E₁) erfaßte, einzelnen Bewegungsphasen (e₁, e₂,...) zugeordnete 3D-Bilddatensätze (p) des Körperorgans (3) enthält, derart, dass derjenige 3D-Bilddatensatz (p) ausgewählt wird, welcher der Bewegungsphase (e₁, e₂,...) bei der Bestimmung der Raumposition (c, r) des medizinischen Instruments (4) und der Referenzprobe (6) zugeordnet ist, und
c) Bestimmung der Position des medizinischen Instruments (4) relativ zu dem Körperorgan (3) durch Umrechnung der Raumposition (c) des medizinischen Instruments (4) mittels der im Raum und im ausgewählten 3D-Bilddatensatz bekannten Positionen der Referenzprobe (6).

2. Verfahren nach Anspruch 1,
**dadurch gekennzeichnet,**
**dass** als Bewegungssignal ein Elektrokardiogramm (E₁, E₂) und/oder ein von der Atembewegung des Patienten (2) abhängiges Atembewegungssignal (A) erfaßt wird.

3. Verfahren nach Anspruch 1,
**dadurch gekennzeichnet,**
**dass** die Bilddatenbank 3D-Bilddatensätze (p) enthält, welche mit einer Bilddatenerfassungseinheit (20), insbesondere einer Magnetresonanzanordnung, einer Röntgeneinrichtung, einer Computertomographieanordnung und/oder einer Ultraschallanordnung erfaßt wurden.

4. Verfahren nach Anspruch 1,
**dadurch gekennzeichnet,**
**dass** auch während der Bestimmung der Raumposition (r) des medizinischen Instruments (4) und der Referenzprobe (6) ein 3D-Bilddatensatz, insbesondere mittels einer Ultraschallanordnung (15), erfaßt und zur Bestimmung der Position des medizinischen Instruments relativ zu dem Körperorgan (3) verwendet und/oder in die Bilddatenbank aufgenommen wird.

5. Verfahren nach Anspruch 1,
**dadurch gekennzeichnet,**
**dass** gleichzeitig zur Bestimmung der Raumposition (c, r) des medizinischen Instruments (4) und der Referenzprobe (6) ein die Atembewegung des Patienten(2) aufzeichnendes Atembewegungssignal (A) gemessen und zur Korrektur bei der Bestimmung der Position des medizinischen Instruments (4) relativ zu dem Körperorgan (3) verwendet wird.

6. Verfahren nach Anspruch 5,
**dadurch gekennzeichnet,**
**dass** die Korrektur derart erfolgt, dass aus einer präoperativ ermittelten Korrekturtabelle, welche Korrekturwerte zur Korrektur des Einflusses der Atembewegung auf die Position des zu untersuchenden Körperorgans (3) in Abhängigkeit von den einzelnen Atembewegungsphasen enthält, der zur aktuell gemessenen Atembewegungsphase gehörige Korrekturwert ausgewählt und damit die Position (c) des medizinischen Instruments (4) im Raum korrigiert wird.

7. Verfahren nach Anspruch 1,
**dadurch gekennzeichnet,**
**dass** aus dem ausgewählten 3D-Bilddatensatz (p) ein Bild des Umgebungsbereichs des medizinischen Instruments (4) bestimmt und auf einer Anzeigevorrichtung (13) dargestellt wird und dass in dieses Bild die Position des medizinischen Instruments (4) eingeblendet wird.

8. Verfahren nach Anspruch 1,
**dadurch gekennzeichnet,**
**dass** die Position der Referenzprobe (6) relativ zu einem 3D-Bilddatensatz **dadurch** ermittelt wird, dass die Referenzprobe (6) bei der Erfassung der 3D-Bilddatensätze (p) mit abgebildet wird oder dass die Position der Referenzprobe (6) relativ zu einer die Bilddatensätze (p) erfassenden Bilddatenerfassungseinheit (20) bestimmt wird.

9. Verfahren nach Anspruch 1,
**dadurch gekennzeichnet,**
**dass** die Referenzprobe (6) fest im Raum angeordnet ist, dass im Verfahrensschritt a die Raumposition einer am Patienten (2) angeordneten Patienten-Referenzprobe (21) ermittelt wird und dass im Verfahrensschritt c bei der Bestimmung der Position des medizinischen Instruments (4) relativ zu dem Körperorgan die im Raum bekannte Position der Patienten-Referenzprobe (21) verwendet wird zur Berücksichtigung von Bewegungen des Patienten (2).

10. Anordnung zur Bestimmung der Position eines in den Körper eines Patienten (2) eingeführten medizinischen Instruments (3) relativ zu einem sich periodisch bewegenden Körperorgan (3) mit
• einer Positionsmeßeinheit (5, 7, 8) zur Bestimmung der Raumposition (c, r) eines in den Körper eingeführten medizinischen Instruments (4) und einer außerhalb des Körpers angeordneten Referenzprobe (6),
• einer Signalmeßeinheit (9, 10; 15, 16) zur Ermittlung eines im Zusammenhang mit der periodischen Bewegung des Körperorgans (3) stehenden periodischen Bewegungssignals (E₂) gleichzeitig zur Bestimmung der Raumposition (c, r) des medizinischen Instruments (4) und der Referenzprobe (6),
• einer Speichereinheit (12) zur Speicherung einer Bilddatenbank, welche präoperativ zeitgleich mit demselben Bewegungssignal (E₁) erfaßte, einzelnen Bewegungsphasen (e₁, e₂,...) zugeordnete 3D-Bilddatensätze (p) des Körperorgans (3) enthält, und mit
• einer Steuer- und Recheneinheit (11) zur Auswahl eines 3D-Bilddatensatzes (p) aus der Bilddatenbank derart, dass derjenige 3D-Bilddatensatz (p) ausgewählt wird, welcher der Bewegungsphase (e₁, e₂, ...) bei der Bestimmung der Raumposition (c, r) des medizinischen Instruments (4) und der Referenzprobe (6) zugeordnet ist, und zur Bestimmung der Position des medizinischen Instruments (4) relativ zu dem Körperorgan (3) durch Umrechnung der Raumposition (c) des medizinischen Instruments (4) mittels der im Raum und im ausgewählten 3D-Bilddatensatz bekannten Positionen der Referenzprobe (6).

11. Anordnung nach Anspruch 10,
**dadurch gekennzeichnet,**
**dass** die Signalmeßeinheit eine Elektrokardiographieeinheit (9, 10) zur Messung eines Elektrokardiogramms (E₂) des Patienten (2) aufweist.

12. Anordnung nach Anspruch 10 oder 11,
**dadurch gekennzeichnet,**
**dass** die Signalmeßeinheit Mittel (15, 16) zur Messung der Atembewegung des Patienten (2) gleichzeitig zur Bestimmung der Raumposition (c, r) des medizinischen Instruments (4) und der Referenzprobe (6) aufweist.

13. Anordnung nach Anspruch 10,
**dadurch gekennzeichnet,**
**dass** die Positionsmeßeinheit (5, 7, 8) außerhalb des Körpers des Patienten (2) bzw. an dem medizinischen Instrument(4) und der Referenzprobe (6) angeordnete elektromagnetische Sendemittel und korrespondierende an dem medizinischen Instrument (4) und der Referenzprobe (6) bzw. außerhalb des Körpers des Patienten (2) angeordnete elektromagnetische Empfangsmittel aufweist.

14. Anordnung nach Anspruch 10,
**dadurch gekennzeichnet,**
**dass** die Referenzprobe (6) ortsfest im Raum angeordnet ist und dass eine am Patienten (2) befestigte Patienten-Referenzprobe (21) vorgesehen ist.

## Claims

1. A method of determining the position of a medical instrument (4), introduced into the body of a patient (2), relative to a periodically moving organ (3) of the body, which method includes the steps of:
a) determining the spatial position (c, r) of the medical instrument (4) and of a reference probe (6) while at the same time a periodic motion signal (E₂) is acquired that relates to the periodic motion of the body organ (3),
b) selecting a 3D image data set (p) from an image data base that contains 3D image data sets (p) of the body organ (3) which have been acquired pre-operatively and simultaneously with the same motion signal (E₁) and are associated with individual motion phases (e₁, e₂, ...), in such a manner that that 3D image data set (p) is selected that is associated with the motion phase (e₁, e₂, ...) during the determination of the spatial position (c, r) of the medical instrument (4) and of the reference probe (6), and
c) determining the position of the medical instrument (4) relative to the body organ (3) by converting the spatial position (c) of the medical instrument (4) by means of the positions of the reference probe (6) which are known in the space and in the selected 3D image data set.

2. A method as claimed in claim 1, **characterized in that** an electrocardiogram (E₁, E₂) and/or a respiratory motion signal (A) which is dependent on the respiratory motion of the patient (2) is acquired as the motion signal.

3. A method as claimed in claim 1, **characterized in that** the image data base contains 3D image data sets (p) which have been acquired by means of an image data acquisition unit (20), notably a magnetic resonance device, an X-ray device, a computed tomography device and/or an ultrasound device.

4. A method as claimed in claim 1, **characterized in that** a 3D image data set is also acquired during the determination of the spatial position (r) of the medical instrument (4) and of the reference probe (6), notably by means of an ultrasound device (15), which image data set is used to determine the position of the medical instrument relative to the body organ (3) and/or is taken up in the image data base.

5. A method as claimed in claim 1, **characterized in that** simultaneously with the determination of the spatial position (c, r) of the medical instrument (4) and of the reference probe (6) there is measured a respiratory motion signal (A) which represents the respiratory motion of the patient (2) and is used for correction purposes during the determination of the position of the medical instrument (4) relative to the body organ (3).

6. A method as claimed in claim 5, **characterized in that** the correction is performed in such a manner that the correction value associated with the actually measured respiratory motion phase is selected from a pre-operatively determined correction table which contains correction values for the correction, in dependence on the individual respiratory motion phases, of the effect of the respiratory motion on the position of the body organ (3) to be examined, the position (c) in space of the medical instrument (4) being corrected on the basis thereof.

7. A method as claimed in claim 1, **characterized in that** an image of the vicinity of the medical instrument (4) is determined from the selected 3D image data set (p) so as to be displayed on a display device (13) and **in that** the position of the medical instrument (4) is superposed on said image.

8. A method as claimed in claim 1, **characterized in that** the position of the reference probe (6) relative to a 3D image data set is determined **in that** the reference probe (6) is also reproduced upon acquisition of the 3D image data sets (p) or **in that** the position of the reference probe (6) is determined relative to an image data acquisition unit (2) acquiring the image data sets (p).

9. A method as claimed in claim 1, **characterized in that** the reference probe (6) has a fixed spatial position, that the spatial position of a patient reference probe (21) arranged on the patient (2) is determined during the step a), and that during the step c) the known spatial position of the patient reference probe (21) is used for determining the position of the medical instrument (4) relative to the body organ in order to take movements of the patient (2) into account.

10. A device for determining the position of a medical instrument (3), introduced into the body of a patient (2), relative to a periodically moving body organ (3), which device includes:
• a position measuring unit (5, 7, 8) for determining the spatial position (c, r) of a medical instrument (4) introduced into the body and of a reference probe (6) arranged outside the body,
• a signal measuring unit (9, 10; 15, 16) for determining a periodic motion signal (E₂), related to the periodic motion of the body organ (3), simultaneously with the determination of the spatial position (c, r) of the medical instrument (4) and of the reference probe (6),
• a storage unit (12) for storing an image data base containing 3D image data sets (p) of the body organ (3) which have been acquired pre-operatively and simultaneously with the same motion signal (E₁) and are associated with individual motion phases (e₁, e₂, ...), and
• a control and arithmetic unit (11) for selecting a 3D image data set (p) from the image data base in such a manner that that 3D image data set (p) is selected that is associated with the motion phase (e₁, e₂) during the determination of the spatial position (c, r) of the medical instrument (4) and of the reference probe (6), and for determining the position of the medical instrument (4) relative to the body organ (3) by converting the spatial position (c) of the medical instrument (4) by means of the positions of the reference probe (6) which are known in the space and in the selected 3D image data set.

11. A device as claimed in claim 10, **characterized in that** a signal-measuring unit includes an electrocardiography unit (9, 10) for measuring an electrocardiogram (E₂) of the patient (2).

12. A device as claimed in claim 10 or 11, **characterized in that** the signal measuring unit includes means (15, 16) for measuring the respiratory motion of the patient (2) simultaneously with the determination of the spatial position (c, r) of the medical instrument (4) and of the reference probe (6).

13. A device as claimed in claim 10, **characterized in that** the position measuring unit (5, 7, 8) includes electromagnetic transmission means which are arranged outside the body of the patient (2), or on the medical instrument (4) and the reference probe (6), as well as corresponding electromagnetic receiving means which are arranged on the medical instrument (4) and the reference probe (6) or outside the body of the patient (2), respectively.

14. A device as claimed in claim 10, **characterized in that** the reference probe (6) is arranged so as to be stationary in space and that there is provided a patient reference probe (21) which is attached to the patient (2).

## Revendications

1. Procédé pour déterminer la position d'un instrument médical (4) introduit dans le corps d'un patient (2) par rapport à un organe corporel (3) se déplaçant périodiquement comportant les étapes suivantes:
a) détermination de la position spatiale (c, r) de l'instrument médical (4) et d'une sonde de référence (6) en cas d'enregistrement simultané d'un signal de mouvement (E₂) périodique par rapport au mouvement périodique de l'organe corporel (3),
b) sélection d'un jeu de données d'image en 3D (p) à partir d'une base de données d'image qui contient des jeux de données d'image en 3D (p) de l'organe corporel (3) attribués à des phases de mouvement individuelles (e₁, e₂, ...) enregistrées à titre préopératoire en même temps que le même signal de mouvement (E₁) de manière à sélectionner le jeu de données d'image en 3D (p) qui est attribué à la phase de mouvement (e₁, e₂, ...) pour la détermination de la position spatiale (c, r) de l'instrument médical (4) et de la sonde de référence (6) et
c) détermination de la position de l'instrument médical (4) par rapport à l'organe du corps (3) par conversion de la position spatiale (c) de l'instrument médical (4) à l'aide des positions de la sonde de référence (6) connues dans l'espace dans le jeu de données d'image en 3D sélectionné.

2. Procédé selon la revendication 1,
**caractérisé en ce**
**qu'**un électrocardiogramme (E₁, E₂) et/ou un signal de mouvement respiratoire (A) dépendant du mouvement respiratoire du patient (2) sont enregistrés comme signal de mouvement.

3. Procédé selon la revendication 1,
**caractérisé en ce**
**que** la base de données d'image contient des jeux de données d'image en 3D (p) qui ont été enregistrés avec une unité d'enregistrement de données d'image (20); en particulier un dispositif de résonance magnétique, un dispositif radiographique, un dispositif de tomographie informatisée et/ou un dispositif aux ultrasons.

4. Procédé selon la revendication 1,
**caractérisé en ce**
**que**, même pendant la détermination de la position spatiale (r) de l'instrument médical (4) et de la sonde de référence (6), un jeu de données d'image en 3D, est enregistré, en particulier à l'aide d'un dispositif aux ultrasons (15) et est utilisé en vue de la détermination de la position de l'instrument médical par rapport à l'organe corporel (3) et/ou est enregistré dans la base de données d'image.

5. Procédé selon la revendication 1,
**caractérisé en ce**
**qu'**en même temps que la détermination de la position spatiale (c, r) de l'instrument médical (4) et de la sonde de référence (6), un signal de mouvement respiratoire (A) enregistrant le mouvement respiratoire du patient (2) est mesuré et utilisé pour la correction en vue de la détermination de la position de l'instrument médical (4) par rapport à l'organe corporel (3).

6. Procédé selon la revendication 5,
**caractérisé en ce**
**que** la correction intervient de telle sorte qu'à partir d'un tableau de correction déterminé à titre préopératoire qui contient des valeurs de correction pour la correction de l'influence du mouvement respiratoire sur la position de l'organe corporel (3) à examiner en fonction des différentes phases de mouvement respiratoire, la valeur de correction appartenant à la phase de mouvement respiratoire actuellement mesurée est sélectionnée et la position (c) de l'instrument médical (4) est dès lors corrigée dans l'espace.

7. Procédé selon la revendication 1,
**caractérisé en ce**
**qu'**à partir du jeu de données d'image en 3D (p) sélectionné, une image des environs de l'instrument médical (4) est déterminée et représentée sur un dispositif d'affichage (13) et que la position de l'instrument médical (4) est intégrée dans cette image.

8. Procédé selon la revendication 1,
**caractérisé en ce**
**que** la position de la sonde de référence (6) est déterminée par rapport à un jeu de données d'image en 3D de telle sorte que la sonde de référence (6) soit représentée lors de l'enregistrement des jeux de données en 3D (p) ou que la position de la sonde de référence (6) soit déterminée par rapport à une unité de saisie des données d'image (2) enregistrant les jeux de données d'image (p).

9. Procédé selon la revendication 1,
**caractérisé en ce**
**que** la sonde de référence (6) est disposée fixement dans l'espace, que, dans l'étape a du procédé, la position spatiale d'une sonde de référence de patient (21) disposée sur le patient (2) est déterminée et que, dans l'étape de procédé (c), la position connue dans l'espace de la sonde de référence du patient (21) est utilisée lors de la détermination de la position de l'instrument médical (4) par rapport à l'organe corporel pour la prise en considération des mouvements du patient (2).

10. Dispositif de détermination d'une position d'un instrument médical (3) introduit dans le corps d'un patient (2) par rapport à un organe corporel se déplaçant périodiquement (3) avec
• une unité de mesure de position (5, 7,8) pour la détermination de la position spatiale (c, r) d'un instrument médical (4) introduit dans le corps et d'une sonde de référence (6) disposée à l'extérieur du corps,
• une unité de mesure du signal (9, 10; 15, 16) pour la détermination d'un signal de mouvement (E₂) périodique en rapport avec le mouvement périodique de l'organe corporel (3) en même temps que la détermination de la position spatiale (c, r) de l'instrument médical (4) et de la sonde de référence (6),
• une unité de mémoire (12) pour l'enregistrement d'une base de données d'images qui contient des jeux de données d'image en 3D (p) de l'organe corporel (3) attribuées aux différentes phases de mouvement (e1, e2, ...) et saisies à titre préopératoire en même temps que le même signal de mouvement (E₁) et
• avec une unité de commande et de calcul (11) pour la sélection d'un jeu de données d'image en 3D (p) à partir de la base de données d'image de manière à sélectionner le jeu de données d'image en 3D (p) qui est attribué à la phase de mouvement (e₁, e₂, ...) pour la détermination de la position spatiale (c, r) de l'instrument médical (4) et de la sonde de référence (6) et pour la détermination de la position de l'instrument médical (4) par rapport à l'organe corporel (3) par conversion de la position spatiale (c) de l'instrument médical (4) à l'aide des positions connues de la sonde de référence (6) dans l'espace et dans le jeu de données d'image en 3D sélectionné.

11. Dispositif selon la revendication 10,
**caractérisé en ce**
**que** l'unité de mesure du signal présente une unité d'électrocardiographie (9, 10) pour la mesure d'un électrocardiogramme (E₂) du patient (2).

12. Dispositif selon l'une des revendications 10 ou 11,
**caractérisé en ce**
**que** l'unité de mesure du signal présente des moyens (15, 16) pour la mesure du mouvement respiratoire du patient (2) en même temps que la détermination de la position spatiale (c, r) de l'instrument médical (4) et de la sonde de référence (6).

13. Dispositif selon la revendication 10,
**caractérisé en ce**
**que** l'unité de mesure de position (5, 7, 8) présente des moyens de transmission électromagnétiques disposés en dehors du corps du patient (2) ou sur l'instrument médical (4) et la sonde de référence (6) et des moyens de réception électromagnétiques corespondants disposés sur l'instrument médical (4) et la sonde de référence (6) ou en dehors du corps du patient (2).

14. Dispositif selon la revendication 10,
**caractérisé en ce**
**que** la sonde de référence (6) est disposée fixement dans l'espace et qu'il est prévu une sonde de référence de patient (21) fixée au patient (2).
